# EUROPÄISCHE PATENTSCHRIFT

(11) **EP 2 827 922 B1**
(45) Veröffentlichungstag und Bekanntmachung des Hinweises auf die Patenterteilung: **14.12.2016**
(21) Anmeldenummer: 13711657.0
(22) Anmeldetag: 20.03.2013
(51) Int. Cl.: A61M 5/48, A61M 5/145

(54) **INJEKTIONSVORRICHTUNG ZUR VERABREICHUNG EINER FLÜSSIGKEIT**
INJECTION DEVICE FOR ADMINISTERING A LIQUID
DISPOSITIF D'INJECTION POUR ADMINISTRER UN LIQUIDE

(30) Priorität: 20.03.2012 DE 102012204394
(43) Veröffentlichungstag der Anmeldung: 28.01.2015
(73) Patentinhaber: B. Braun Melsungen AG, 34212 Melsungen (DE)
(72) Erfinder: WEISS, Andre, 34302 Guxhagen (DE); WIEGEL, Heinz, 36211 Ahlheim (DE)
(74) Vertreter: dompatent von Kreisler Selting Werner - Partnerschaft von Patent- und Rechtsanwälten mbB
(86) Internationale Anmeldenummer: PCT/EP2013/055811
(87) Internationale Veröffentlichungsnummer: WO 2013/139852

(56) Entgegenhaltungen:
- EP-A1- 1 813 300
- WO-A1-90/10468
- GB-A- 2 463 051
- US-A- 4 731 058
- US-A- 4 759 750
- US-A- 5 808 203

## Beschreibung

Die Erfindung betrifft eine Injektionsvorrichtung zur Verabreichung einer Flüssigkeit.

Bekannt sind Injektionsvorrichtungen mit einem die Flüssigkeit enthaltenden Zylinder und mit einem Auslass für die Flüssigkeit. Ein in dem Zylinder angeordneter Kolben presst die Flüssigkeit bei Verschieben des Kolbens aus dem Auslass heraus. Dazu wird auf den Kolben eine Kraft (Betätigungsdruck) aufgebracht, durch die ein Druck (Flüssigkeitsdruck) in der im Zylinder befindlichen Flüssigkeit aufgebaut wird. Derartige Injektionsvorrichtungen finden typischerweise im medizinischen Bereich Anwendung, beispielsweise als Spritzen zur Injektion von Medikamenten oder Anästhetika. Hierbei kann mit dem Zylinderauslass eine hohle Spritzennadel, ein Katheter oder anderweitiger Schlauch zur Fortleitung der Flüssigkeit verbunden sein. Die Injektionsvorrichtung kann aber auch zur Befüllung von Behältern dienen.

Bei diesen Anwendungen ist oftmals der Druck, mit welchem die Flüssigkeit aus dem Zylinder herausgepresst wird, von besonderer Bedeutung. Bei der Injektion von Medikamenten oder Anästhetika in einen Patienten kann ein zu hoher Druck zu physiologischen Schäden führen. Beispielsweise können Blutgefäße, Gewebe und/oder Nerven durch einen zu hohen Druck beschädigt werden. Insbesondere bei intraneuraler Injektion von Anästhetika können Nerven bei einem Druck von mehr als 20 psi, d. h. von mehr als ungefähr 1,4 bar, beschädigt werden. Bei anderen Anwendungen besteht die Gefahr einer Beschädigung oder Zerstörung von Filtern oder eines mit dem Auslass verbundenen Leitungssystems, wie beispielsweise einem Ballonkatheter zur Applikation von Stents.

Der Erfindung liegt die Aufgabe zugrunde, eine Injektionsvorrichtung mit einer Überdrucksicherung zu schaffen.

Die Erfindung wird definiert durch die Merkmale von Patentanspruch 1.

Demnach weist die Injektionsvorrichtung eine Überdrucksicherung mit einem den Flüssigkeitsdruck indirekt erfassenden Sensor und mit einem den Flüssigkeitsdruck der aus dem Auslass austretenden Flüssigkeit reduzierenden Aktor, wobei der Aktor eine weitere Erhöhung des Flüssigkeitsdrucks in Abhängigkeit des durch den Sensor erfassten Flüssigkeitsdrucks verhindert. Der Sensor kann den Flüssigkeitsdruck indirekt erfassen, indem der auf den Kolben wirkende Betätigungsdruck direkt erfasst wird. Hierbei wirkt der Betätigungsdruck auf den Kolben, um diesen zu verschieben und den erforderlichen Flüssigkeitsdruck zu erzeugen, durch den die Flüssigkeit aus dem Auslass herausgepresst wird. Ein zu hoher Betätigungsdruck könnte in einem zu hohen Flüssigkeitsdruck resultieren. Der Sensor erfasst einen zu hohen Flüssigkeitsdruck indirekt und veranlasst den Aktor, eine weitere Erhöhung des Flüssigkeitsdrucks zu verhindern. Die Injektionsvorrichtung ist also mit einer integrierten Überdrucksicherung versehen, die autark, d. h. unabhängig von den Fähigkeiten des Bedieners oder von äußeren Überwachungseinrichtungen, einen zu hohen Druck innerhalb des Spritzenzylinders erfasst und automatisch eine weitere Druckerhöhung verhindert.

Hierbei ist der Sensor ein zwischen dem Kolben und einem auf den Kolben einwirkenden Stempel angeordnetes Federelement. Der Stempel wird zum Verabreichen der Flüssigkeit in den Zylinder eingeschoben und übt dabei den auf den Kolben wirkenden Betätigungsdruck aus. Das Federelement erfasst auf technisch einfache Weise einen zu hohen auf den Kolben wirkenden Druck. Der Flüssigkeitsdruck wird von dem Federelement indirekt erfasst.

Der Aktor weist eine die Vorschubgeschwindigkeit des Kolbens gegenüber dem Zylinder reduzierende Bremsvorrichtung auf. Die Bremsvorrichtung wird durch den Sensor ausgelöst. Dieser kann beispielsweise durch das Federelement realisiert werden. Von besonderem Vorteil ist es, wenn die Bremsvorrichtung an dem Kolben angeordnete und von dem Aktor gegen die Innenwand des Zylinders ausgelenkte Bremselemente aufweist. Hierbei kann das Federelement zwei starr mit dem Kolben verbundene und elastisch federnde Biegefedern, insbesondere Federschenkel aufweisen, die die Bremselemente des Aktors bilden. Bei Überschreiten einer geeigneten Federkraft werden die Schenkel und somit die Bremselemente des Federelements gegen die Innenwand des Zylinders ausgelenkt. Insbesondere in Verbindung mit dem Federelement ist dabei eine technisch einfache, haltbare und störunanfällige Überdrucksicherung geschaffen. Bei Überschreiten eines kritischen Betätigungs- bzw. Flüssigkeitsdrucks wird durch Blockieren des Kolbens eine weitere Erhöhung des Flüssigkeitsdrucks verhindert. Bei anschließender Verringerung des Betätigungs- bzw. Flüssigkeitsdrucks kann der Sensor die Bremselemente automatisch oder manuell von Innen von der

Innenwand des Zylinders wegführen, so dass die Bremskraft vermindert wird und der Kolben nicht länger blockiert wird. Die Überdrucksicherung ist also reversibel.

Alternativ ist denkbar, dass der Sensor ein Drucksensor, beispielsweise ein Manometer zur Messung des Flüssigkeitsdrucks in dem Zylinder oder hinter der Austrittsöffnung des Auslasses aufweist. Bei dem Aktor kann es sich alternativ um ein Ventil, beispielsweise um ein Überdruckventil an dem Zylinder handeln. Das Ventil lässt bei Überschreiten des kritischen Drucks Flüssigkeit aus dem Zylinder entweichen. Bei dem Ventil kann es sich auch um ein dem Auslass nachgeschaltetes Druckminderventil oder um ein druckgesteuertes Ventil handeln, welches bei Überschreiten des kritischen Drucks den Auslass oder eine mit dem Auslass verbundene Flüssigkeitsleitung verschließt.

Die Überdrucksicherung und insbesondere der Sensor und der Aktor weisen vorzugsweise mindestens einen metallischen Werkstoff, wie z. B. Stahl, medizinischen Edelstahl, Messing, Aluminium, Gold, Silber und/oder Platin auf. Alternativ oder ergänzend können auch Glas, keramische Elemente wie z. B. Prozellanstopfen und/oder Kunststoffe Anwendung finden. Materialkombinationen wie z. B. ein Halter aus einem Stahl mit einem Glaseinsatz für Spritzenzylinder und eine Kolbenstange aus Stahl mit Kunststoffdichtringen sind ebenfalls denkbar.

Im folgenden werden anhand der Figuren zwei Ausführungsbeispiele der Erfindung näher erläutert. Es zeigen:
- Fig. 1: eine Explosionsansicht der erfindungsgemäßen Injektionsvorrichtung nach dem ersten Ausführungsbeispiel,
- Fig. 2: ein Detail gemäß Fig. 1,
- Fig. 3: die Ansicht nach Fig. 2 im betätigten Zustand und
- Fig. 4: das der Ansicht gemäß den Fign. 2 und 3 entsprechende Detail des zweiten Ausführungsbeispiels.

In den Figuren sind der Zylinder 12, der Kolben 14, die Überdrucksicherung 16 und der Stempel 18 des dargestellten Ausführungsbeispiels der Injektionsvorrichtung 10 gezeigt. Der Zylinder 12 ist mit einem stirnseitigen Auslass 20 versehen. Zur Veranschaulichung ist der Auslass 20 als offene Stirnseite des Zylinders 12 dargestellt. Typischerweise wird es sich bei dem Auslass 20 um einen herkömmlichen Auslass eines Spritzenzylinders handeln. Der Auslass 20 soll auf technisch einfache und herkömmliche Weise mit einer Flüssigkeitsfortleitung, wie beispielsweise einem Katheter oder einer Spritzennadel verbindbar sein.

Das dem Auslass 20 gegenüberliegende stirnseitige Ende 22 des Zylinders 12 ist ebenfalls ein offenes Ende, in welches der Kolben 14 und der Stempel 18 zur Betätigung des Kolbens 14 eingeschoben werden. Zwischen dem Kolben 14 und dem Stempel 18 ist die Überdrucksicherung 16 angeordnet und jeweils mit dem Kolben 14 und mit dem Stempel 18 verbunden.

Die Überdrucksicherung 16 besteht aus einem Sensor 24 und einem Aktor 26. Der Sensor 24 ist ein Federelement, welches aus zwei Federschenkeln 27 besteht, die jeweils mit der dem Kolben 14 zugewandten Stirnseite des Stempels 18 starr verbunden sind. Die Federschenkel 27 sind jeweils elastisch federnd ausgebildet und bilden ein V. Die von dem Kolben 14 abgewandten Enden der Federschenkel 27 sind jeweils gelenkig mit einem Filmscharnier 28 verbunden. Jedes Filmscharnier 28 ist gelenkig mit einem mittig an der proximalen Stirnseite des Stempels 18 ausgebildeten Sockel 29 verbunden. Der Aktor 26 enthält eine Bremsvorrichtung, die aus zwei Bremselementen 30 besteht, die jeweils an den äußeren, dem Kolben 14 gegenüberliegenden Enden der Federschenkel 27 ausgebildet sind. Jedes Bremselement 30 ist seitlich nach außen in Richtung auf die Innenseite der Kolbenwand gerichtet. Der Aktor 26 besteht aus dem Federelement mit den beiden Bremselementen 30 sowie aus den Filmscharnieren 28.

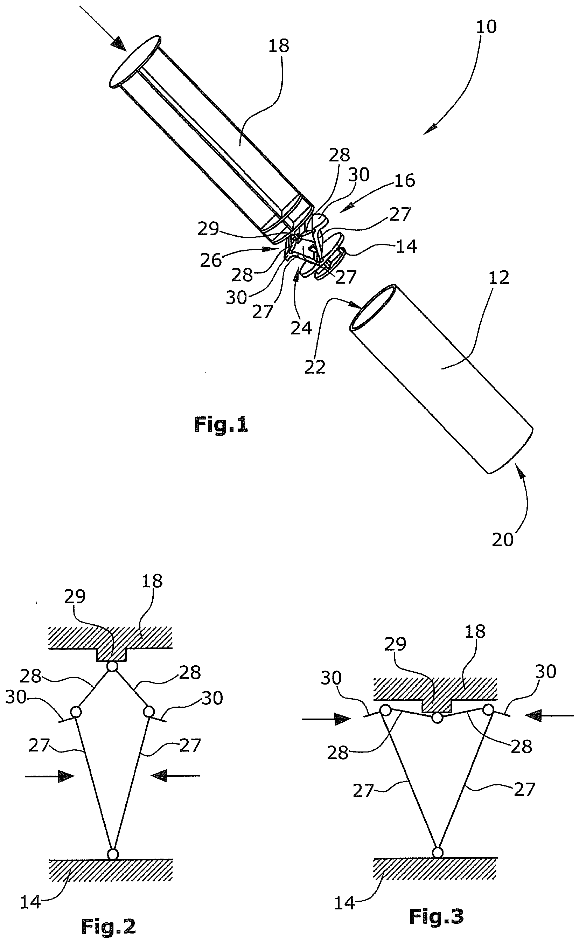

Das aus den Federschenkeln 27 gebildete Federelement weist eine solche Federkonstante auf, dass bei Überschreiten eines kritischen Betätigungsdrucks auf den Stempel 18 von beispielsweise ungefähr 20 psi, d. h. ungefähr 1,4 bar, das Federelement aktiviert wird. Bei der Aktivierung des Federelements drücken die Filmscharniere 28, wie in den Figuren 2 und 3 dargestellt, die Federschenkel 27 nach außen. Bei Überstrecken der Filmscharniere 28 presst die in den Figuren 2 und 3 durch die beiden Pfeile dargestellte Federkraft die Filmscharniere 28 nach innen und gegen den Sockel 29. In der Figur 3 ist das Federelement aktiviert und hält die Filmscharniere 28 in einer stabilen Lage, in der die Bremselemente 30 an den äußeren Enden der Federschenkel 27 gegen die Innenwand des Zylinders 12 drücken. Hierbei werden die Filmscharniere 28 derart verschwenkt, dass der auf den Stempel 18 aus Richtung des in der Figur dargestellten Pfeils in Axialrichtung des Zylinders 12 wirkende Teil des Betätigungsdrucks von den Filmscharnieren 28 radial nach außen auf die beiden Bremselemente 30 übertragen wird. Die Bremselemente 30 werden dadurch radial nach außen gegen die Innenwand des Zylinders 12 gepresst. Auf diese Weise wird nur ein maximal zulässiger Druck von dem Stempel 18 auf den Kolben 14 übertragen, während ein überschüssiger Druck von dem Sensor 24 erfasst wird und über den Aktor 26 in eine Bremskraft zum Abbremsen und Blockieren des Kolbens 14 umgewandelt wird. Der Kolben 14 wird durch Reibung zwischen den Bremselementen 30 und der Innenwand des Zylinders 12 blockiert.

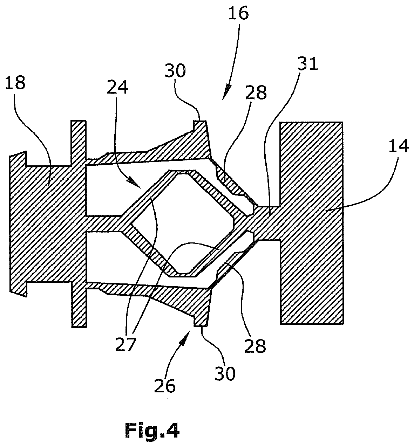

Das zweite Ausführungsbeispiel gemäß Fig. 4 unterscheidet sich von dem ersten Ausführungsbeispiel durch den Aufbau der Überdrucksicherung 16. Die beiden Bremselemente 30 sind jeweils über ein Filmscharnier 28 mit einem Sockel 31 an dem Kolben 14 verbunden. An dem jeweils anderen Ende sind die Bremselemente 30 jeweils gelenkig mit dem Stempel 18 verbunden. Der Sensor 24 der Überdrucksicherung 16 ist ein Federelement, welches aus zwei Federschenkeln 27 besteht. Die Federschenkel 27 sind an ihren Enden miteinander verbunden und bilden ein O oder ein Trapez, wie in Fig. 4 dargestellt. Die miteinander verbundenen Enden der Federschenkel 27 sind auf der einen Seite mit dem Sockel 31 an dem Kolben 14 und auf der anderen Seite mit dem Stempel 18 verbunden. Die Federschenkel 27 sind zwischen den beiden Bremselementen 30 und den zugehörigen Filmscharnieren 31 angeordnet. Während also bei dem ersten Ausführungsbeispiel ein Federschenkel 27 und ein Filmscharnier 28 in Reihe hintereinander angeordnet sind, sind die Federschenkel 27 und die Filmscharniere 28 bei dem zweiten Ausführungsbeispiel nebeneinander angeordnet, wobei jeweils ein Bremselement 30 und ein Filmscharnier 28 in Reihe hintereinander angeordnet sind.

Ein auf den Stempel 18 wirkender überschüssiger Druck wird von dem Sensor 24 in Form der Federschenkel 27 erfasst und drückt die Federschenkel 27 entgegen der Federkraftwirkung auseinander. Dabei werden die Bremselemente 30 von den Filmscharnieren 28 ebenfalls nach außen und gegen die Innenwand des in Fig. 4 nicht dargestellten Zylinders 12 gedrückt. Der aus den Bremselementen 30 und den Filmscharnieren 28 bestehende Aktor 26 wandelt den auf den Stempel 18 wirkenden überschüssigen Druck in eine Bremskraft zum Abbremsen und Blockieren des Kolbens 14 um.

Bei einem weiteren, in den Figuren nicht dargestellten Ausführungsbeispiel kann der Sensor ein Manometer zur Messung des Flüssigkeitsdrucks innerhalb des Zylinders 12 oder in einer mit dem Auslass 20 verbundenen Flüssigkeitsleitung sein. Der Aktor kann ein Ventil oder eine bei Überdruck berstende Membran zur Fortleitung überschüssigen Flüssigkeitsdrucks sein.

## Patentansprüche

1. Injektionsvorrichtung (10) zur Verabreichung einer Flüssigkeit, mit
einem Zylinder (12) mit einem Auslass (20) für die Flüssigkeit und
einem in dem Zylinder (12) angeordneten Kolben (14), der dazu ausgebildet ist, durch Verschieben in dem Zylinder (12) die Flüssigkeit aus dem Auslass (20) herauszupressen,
wobei die Injektionsvorrichtung (10) eine Überdrucksicherung (16) mit einem Sensor (24), der den Flüssigkeitsdruck in dem Zylinder (12) indirekt erfasst, und
der Sensor (24) ein Federelement ist, das zwischen dem Kolben (14) und einem auf den Kolben wirkenden Stempel (18) angeordnet ist, **dadurch gekennzeichnet dass** die Injektionsvorrichtung (10) einen Aktor (26) aufweist und
dass der Aktor (26) eine die Vorschubgeschwindigkeit des Kolbens (14) in dem Zylinder (12) reduzierende Bremsvorrichtung aufweist, um in Abhängigkeit von dem erfassten Flüssigkeitsdruck eine weitere Erhöhung des Flüssigkeitsdrucks der aus dem Auslass (20) herausgepressten Flüssigkeit zu verhindern.

2. Injektionsvorrichtung (10) nach Anspruch 1, **dadurch gekennzeichnet, dass** das Federelement aus zwei mit dem Kolben (14) verbundenen Biegefedern, insbesondere Federschenkeln (27), besteht.

3. Injektionsvorrichtung nach Anspruch 1 oder 2, **dadurch gekennzeichnet, dass** der Aktor (26) mindestens ein mit dem Stempel (18) verbundenes Filmscharnier (28) aufweist, welches gelenkig mit dem Federelement verbunden ist.

4. Injektionsvorrichtung nach Anspruch 3, **dadurch gekennzeichnet, dass** jedes Filmscharnier (28) gelenkig mit einem von einer Stirnseite des Stempels (18) abstehenden Sockel gelenkig verbunden ist.

5. Injektionsvorrichtung nach Anspruch 1 oder 2, **dadurch gekennzeichnet, dass** der Aktor (26) mindestens zwei Bremselemente (30) aufweist, zwischen denen das Federelement angeordnet ist.

6. Injektionsvorrichtung nach Anspruch 5, **dadurch gekennzeichnet, dass** jedes Bremselement (30) an einem Ende gelenkig mit dem Stempel (18) oder dem Kolben (14) und an einem gegenüberliegenden Ende mit einem Filmscharnier (28) verbunden ist, welches mit dem Kolben (14) bzw. dem Stempel (18) verbunden ist.

7. Injektionsvorrichtung nach Anspruch 5 oder 6, **dadurch gekennzeichnet, dass** das Federelement bei Überschreiten dessen Federkraft nach außen gegen die Bremselemente (30) ausgelenkt wird und diese nach außen drückt.

8. Injektionsvorrichtung (10) nach einem der vorhergehenden Ansprüche, **dadurch gekennzeichnet, dass** der Sensor (24) den zur Verschiebung des Kolbens (14) aufgebrachten Betätigungsdruck erfasst.

9. Injektionsvorrichtung (10) nach Anspruch 1, **dadurch gekennzeichnet, dass** die Bremsvorrichtung von dem Aktor (26) gegen die Innenwand des Zylinders (12) auslenkbare Bremselemente (30) aufweist.

## Claims

1. An injection device (10) for administering a liquid, comprising
a barrel (12) including an outlet (20) for the liquid, and
a piston (14) arranged in said barrel (12) and adapted to force the liquid out of said outlet (20) by displacement in said barrel (12),
wherein said injection device (10) includes a pressure relief means (16) having a sensor (24) directly or indirectly detecting the liquid pressure in said barrel (12), and
the sensor (24) is a spring element arranged between the piston (14) and a plunger (18) acting upon said piston,
**characterized in that**
said injection device (10) includes an actuator (26) and
**in that** said actuator (26) comprises a braking device reducing the advancing rate of the piston (14) in the barrel (12) to prevent a further increase in the liquid pressure of the liquid forced out of said outlet (20) depending on the detected liquid pressure.

2. The injection device (10) according to claim 1, **characterized in that** the spring element is made up of two flexible springs, in particular spring legs (27), connected with the piston (14).

3. The injection device according to claim 1 or 2, **characterized in that** the actuator (26) comprises at least one living hinge (28) connected with the plunger (18) and articulated to the spring element.

4. The injection device according to claim 3, **characterized in that** each living hinge (28) is articulated to a base projecting from a front end of the plunger (18).

5. The injection device according to claim 1 or 2, **characterized in that** the actuator (26) comprises at least two braking elements (30) between which the spring element is arranged.

6. The injection device according to claim 5, **characterized in that** each braking element (30) is articulated at one end to the plunger (18) or the piston (14) and at an opposite end to a living hinge (28) which is connected with said piston (14) and/or said plunger (18).

7. The injection device according to claim 5 or 6, **characterized in that** the spring element is deflected outwards against the braking elements (30) and presses the latter outwards when its spring force is exceeded.

8. The injection device (10) according to any one of the preceding claims, **characterized in that** the sensor (24) detects the actuation pressure applied for displacing the piston (14).

9. The injection device (10) according to claim 1, **characterized in that** the braking device comprises braking elements (30) adapted to be deflected against the inner wall of the barrel (12) by the actuator (26).

## Revendications

1. Dispositif d'injection (10) pour l'administration d'un liquide, avec un cylindre (12) avec une sortie (20) pour le liquide et un piston (14) disposé dans le cylindre (12), qui est conçu pour expulser, par coulissement dans le cylindre (12), le liquide hors de la sortie (20),
le dispositif d'injection (10) comprenant une sécurité anti-surpression (16) avec un capteur (24) qui mesure indirectement la pression de liquide dans le cylindre (12) et
le capteur (24) est un élément à ressort qui est disposé entre le piston (14) et un poinçon (18) agissant sur le piston,
**caractérisé en ce que** le dispositif d'injection (10) comprend un actionneur et
**en ce que** l'actionneur (26) comprend un dispositif de freinage réduisant la vitesse d'avance du piston (14) dans le cylindre (12), afin d'empêcher, en fonction de la pression de liquide mesurée, une augmentation supplémentaire de la pression du liquide expulsé hors de la sortie (20).

2. Dispositif d'injection (10) selon la revendication 1, **caractérisé en ce que** l'élément à ressort est constitué de ressorts de flexion reliés avec le piston (14), plus particulièrement de branches de ressorts (27).

3. Dispositif d'injection (10) selon la revendication 1 ou 2, **caractérisé en ce que** l'actionneur (26) comprend au moins une charnière à film (28) reliée avec le poinçon (18), qui est reliée de manière articulée avec l'élément à ressort.

4. Dispositif d'injection (10) selon la revendication 3, **caractérisé en ce que** chaque charnière à film (28) est reliée de manière articulée avec un socle éloigné d'un côté frontal du poinçon (18).

5. Dispositif d'injection (10) selon la revendication 1 ou 2, **caractérisé en ce que** l'actionneur (26) comprend au moins deux éléments de freinage (30) entre

6. Dispositif d'injection (10) selon la revendication 5, **caractérisé en ce que** chaque élément de freinage (30) est relié, à une extrémité, de manière articulée avec le poinçon (18) ou le piston (14) et, à une extrémité opposée, avec une charnière à film (28), qui est reliée avec le piston (14) ou le poinçon (18).

7. Dispositif d'injection (10) selon la revendication 5 ou 6, **caractérisé en ce que** l'élément à ressort est dévié, lors d'un dépassement de sa force élastique, vers l'extérieur contre les éléments de freinage (30) et comprime ceux-ci vers l'extérieur.

8. Dispositif d'injection (10) selon l'une des revendications précédentes, **caractérisé en ce que** le capteur (24) mesure la pression d'actionnement exercée pour le coulissement du piston (14).

9. Dispositif d'injection (10) selon la revendication 1, **caractérisé en ce que** le dispositif de freinage comprend des éléments de freinage (30) pouvant être déviés de l'actionneur (26) contre la paroi interne du cylindre (12).
